# EUROPEAN PATENT APPLICATION

(11) **EP 4 588 494 A1**
(43) Date of publication of application: **23.07.2025**
(21) Application number: 22958358.8
(22) Date of filing: 13.09.2022
(51) Int. Cl.: A61M 5/142, G16H 40/60, G16H 20/17

(54) **DRUG INFUSION SYSTEM WITH GRAPHICAL USER INTERFACE**

(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2022/118490
(87) International publication number: WO 2024/055164

(57) **Abstract**

The invention discloses a drug delivery system with graphical user interface comprises detection device, for detecting the blood glucose level of the user; an infusion device, for infusing a drug into the user's body; and a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein the insulin information display area comprising insulin infusion status information, the graphical user interface displays the insulin infusion status information in a graphical and/or textual manner, to facilitate the user to intuitively know the insulin infusion status information and blood glucose information at that time.

## Description

### TECHNICAL FIELD

The present invention mainly relates to the field of medical instruments, in particular to a drug delivery system with graphical user interface.

### BACKGROUND

In a healthy person, the pancreas can automatically monitor the amount of glucose in the blood and automatically secrete the required dosage of insulin/glucagon. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods can continuously detect blood glucose and send the blood glucose data to the remote device in real-time for the user to view. This detection method is called Continuous Glucose Monitoring (CGM), which requires the detection device to be attached to the surface of the patient's skin, and the sensor carried by the device to be inserted into the interstitial fluid for detection. According to the blood glucose (BG) level, the control device of the delivery system calculates the amount of insulin required for the current or future period, and the infusion device delivers insulin subcutaneously according to the insulin infusion strategy input by the control device or the user, thus forming a closed-loop or semi-closed-loop artificial pancreas.

Currently, due to the miniaturization trend of devices, the area on the infusion device and/or control device for displaying blood glucose or insulin information, or the area for the user to input, is also getting smaller and smaller, resulting in the user not being able to intuitively know the insulin infusion status information and/or blood glucose information they need to pay attention to, and the user not being able to operate it autonomously and conveniently, resulting in a poor user experience.

Therefore, in the prior art, there is an urgent need for a drug delivery system for displaying drug infusion information that makes it easy for users to know insulin infusion status information.

### BRIEF SUMMARY OF THE INVENTION

The invention discloses a drug delivery system with graphical user interface, comprising a detection device, an infusion device, and a control device, the control device provided with a graphical user interface, the graphical user interface comprising a main screen, the main screen comprising an insulin information display area and a blood glucose information display area, wherein the insulin information display area comprising insulin infusion status information, the graphical user interface displays the insulin infusion status information in a graphical and/or textual manner, to facilitate the user to intuitively know the insulin infusion status information and blood glucose information at that time.

The invention discloses a drug delivery system with graphical user interface that includes a detection device, for detecting the blood glucose level of the user; an infusion device, for infusing a drug into the user's body; and a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein the insulin information display area comprising insulin infusion status information, the graphical user interface displays the insulin infusion status information in a graphic and/or text manner.

According to one aspect of the present invention, the graphic is a ring with at least one color indicating the type and the progress of the insulin infusion, the text comprising an abbreviation of the type of infusion and the rate or the progress of the infusion.

According to one aspect of the present invention, the elastic seal and the security base body are integrally formed by injection molding.

According to one aspect of the present invention, the blood glucose information includes a blood glucose graphic display area and a status display area of the detection device.

According to one aspect of the present invention, the status display area of the detection device area is set inside the ring.

According to one aspect of the present invention, the detection device status display area displays information that the detection device is in a general detection state or a special detection state.

According to one aspect of the present invention, when the detection device is in the general detection state, the detection device status display area shows the blood glucose reading of the latest moment.

According to one aspect of the present invention, when the detection device is in the special detection state, the detection device status display area shows a text message with a specific meaning to alert the user that an abnormal blood glucose value was detected by the detection device or there is an abnormal in the detection device.

According to one aspect of the present invention, the blood glucose graphical display area includes a blood glucose reading curve or blood glucose reading discrete points over a period of time, and the normal blood glucose value area.

According to one aspect of the present invention, when the detection device is in the general detection state, the detection device status display area further includes a blood glucose trend display area or standardized lines for high blood glucose values and low blood glucose values.

According to one aspect of the present invention, the blood glucose trend display area is indicated by indicator arrows with different color, direction and number.

According to one aspect of the present invention, the blood glucose trend display area is set in the ring.

According to one aspect of the present invention, the main screen further includes a calibration display area of the detection device.

According to one aspect of the present invention, the calibration display area dynamically displays the calibration status of the detection device over time.

According to one aspect of the present invention, the main screen further includes a blood glucose data recovery icon, the blood glucose data recovery icon appears during the blood glucose data recovery and disappears when the blood glucose data is fully recovered.

According to one aspect of the present invention, the main screen further includes a suspend infusion function icon, for displaying the state of hypoglycemia suspend infusion function or predicted hypoglycemia suspend infusion function in the drug infusion system.

According to one aspect of the present invention, the main screen further includes an alert status icon.

According to one aspect of the present invention, the detection device and the infusion device are provided in separate components.

According to one aspect of the present invention, the detection device and the infusion device are provided in same component.

The invention further discloses a drug delivery system with graphical user interface that includes a detection device, for detecting the blood glucose level of the user; an infusion device, for infusing a drug into the user's body; and a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein to access other screens by sliding on the main screen.

According to one aspect of the present invention, the sliding method includes the direction and/or the number of sliding, sliding in different directions and/or number of times to make the home screen to access other different screens.

According to one aspect of the present invention, the graphical user interface returns to the home screen from the other different screens by sliding.

According to one aspect of the present invention, the sliding method to access other different screens is related to the sliding method to return to the home screen from the other different screens.

According to one aspect of the present invention, the sliding method to access other different screens and the sliding method to return to the home screen from the other different screens has the same sliding number of times but different sliding directions.

According to one aspect of the present invention, the graphical user interface returns to the home screen from the other different screens by a particular way.

According to one aspect of the present invention, the user interface is set with a touch-sensitive button, the particular way is touching the touch-sensitive button to return to the home screen.

According to one aspect of the present invention, the particular way is tapping the user interface in succession to return to the home screen.

According to one aspect of the present invention, the particular way is sliding in a certain direction and number of times to return to the home screen.

According to one aspect of the present invention, the control device further includes a solid button, the particular way is pressing the solid button to return to the home screen.

According to one aspect of the present invention, the insulin information display area includes insulin infusion status information, IOB information and remaining insulin information in the infusion device, the user interface displaying the insulin infusion status information in a graphic and/or text manner.

According to one aspect of the present invention, the blood glucose information includes a blood glucose graphic display area and a status display area of the detection device.

According to one aspect of the present invention, the detection device status display area displays information that the detection device is in a general detection state or a special detection state.

According to one aspect of the present invention, the blood glucose graphical display area includes a blood glucose reading curve or blood glucose reading discrete points over a period of time, and the normal blood glucose value area or the standard lines for high blood glucose value and low blood glucose value.

According to one aspect of the present invention, when the detection device is in the general detection state, the detection device status display area further includes a blood glucose trend display area.

According to one aspect of the present invention, the main screen further includes a calibration display area of the detection device.

According to one aspect of the present invention, the main screen further includes a blood glucose data recovery icon, the blood glucose data recovery icon appears during the blood glucose data recovery and disappears when the blood glucose data is fully recovered.

According to one aspect of the present invention, the main screen further includes a suspend infusion function icon, for displaying the state of hypoglycemia suspend infusion function or predicted hypoglycemia suspend infusion function in the drug infusion system.

According to one aspect of the present invention, the main screen further includes an alert status icon.

The invention further discloses a drug delivery system with graphical user interface that includes a detection device, for detecting the blood glucose level of the user; an infusion device, for infusing a drug into the user's body; and a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein the insulin information display area includes insulin infusion status information, the blood glucose information display area includes a blood glucose graphical display area that graphically displays the user's blood glucose level in a first time period, and by user input, the graphical user interface displays the user's blood glucose level in other time periods.

According to one aspect of the present invention, the blood glucose level in other time periods is displayed on the main screen.

According to one aspect of the present invention, by the user input, a time period selection area is displayed on the main screen, by selecting different time periods, the blood glucose graphical display area displays blood glucose level in the same time period.

According to one aspect of the present invention, the blood glucose level in other time periods is displayed on an expanded blood glucose graphical display area.

According to one aspect of the present invention, the expanded blood glucose graphical display area at least partially cover the main screen.

According to one aspect of the present invention, the expanded blood glucose graphical display area includes at least a time period selection area and a blood glucose graphical display area.

According to one aspect of the present invention, the expanded blood glucose graphical display area further includes a blood glucose text display area, for displaying the information about the blood glucose value at the selected moment or special detection status information.

According to one aspect of the present invention, in response to another user input, the blood glucose graphical display area displays the information on blood glucose values at the selected moment or special detection status information.

According to one aspect of the present invention, the blood glucose graphic display area further includes an event indication icon to indicate the time the event occurs.

According to one aspect of the present invention, by another user input, the detailed blood glucose information interface is accessed from the blood glucose graphic display area.

According to one aspect of the present invention, the detailed blood glucose information interface at least includes basic statistics and distribution statistics.

According to one aspect of the present invention, the blood glucose information display area further includes the status display area of the detection device.

According to one aspect of the present invention, the detection device status display area displays information that the detection device is in a general detection state or a special detection state.

According to one aspect of the present invention, when the detection device is in the general detection state, the detection device status display area shows the blood glucose reading of the latest moment.

According to one aspect of the present invention, when the detection device is in the special detection state, the detection device status display area shows a text message with a specific meaning to alert the user that an abnormal blood glucose value was detected by the detection device or there is an abnormal in the detection device.

According to one aspect of the present invention, the user interface displays the insulin infusion status information in a graphical and/or textual manner.

According to one aspect of the present invention, the graphic is a ring with at least one color indicating the type and the progress of the insulin infusion, the text comprising an abbreviation of the type of infusion and the rate or the progress of the infusion.

According to one aspect of the present invention, the status display area of the detection device area is set inside the ring.

According to one aspect of the present invention, when the detection device is in the general detection state, the detection device status display area further includes a blood glucose trend display area.

According to one aspect of the present invention, the blood glucose trend display area is indicated by indicator arrows with different color, direction and number.

The invention further discloses a drug delivery system with graphical user interface that includes a detection device, for detecting the blood glucose level of the user; a control device, wirelessly communicated with the infusion device, the control device controlling the drug delivery of the infusion device, and the control device provided with a graphical user interface, the graphical user interface comprising a main screen, the main screen comprising an insulin information display area, wherein the user interface displaying the insulin infusion status information in a graphic and/or text manner.

According to one aspect of the present invention, the graphic is a ring with at least one color indicating the type and the progress of the insulin infusion, the text comprising an abbreviation of the type of infusion and the rate or the progress of the infusion.

According to one aspect of the present invention, when the detection device is in the special detection state, the detection device status display area shows a text message with a specific meaning to alert the user that an abnormal blood glucose value was detected by the detection device or there is an abnormal in the detection device.

According to one aspect of the present invention, the main screen also includes a graphic display area of insulin infusion information, which displays insulin infusion information in graphical manner for a period of time.

According to one aspect of the present invention, in response to a first user input, the insulin infusion information graphic display area displays insulin infusion information at a selected moment.

According to one aspect of the present invention, in response to a second user input, the insulin infusion information graphic display area is accessed to an expanded insulin infusion information graphic display area.

According to one aspect of the present invention, the expanded insulin infusion information graphical display area includes at least a time period selection area and an insulin infusion information graphical display area.

According to one aspect of the present invention, the expanded blood glucose graphical display area further includes an insulin infusion information text display area.

According to one aspect of the present invention, the insulin infusion information graphic display area further includes an event indication icon to indicate the time the event occurs.

According to one aspect of the present invention, by a third user input, the insulin infusion information graphic display area is accessed to a detailed insulin infusion information interface.

According to one aspect of the present invention, the detailed insulin infusion information interface at least includes basic statistics.

According to one aspect of the present invention, in response to a fourth user input, a time period selection area is displayed on the main screen, by selecting different time periods to view the insulin infusion information level in the corresponding different time periods.

According to one aspect of the present invention, in response to a fifth user input, the detailed insulin infusion information interface is accessed from the insulin infusion information graphic display area.

According to one aspect of the present invention, the detailed insulin infusion information interface at least includes basic statistics.

According to one aspect of the present invention, the main screen at least further includes one or more IOB icon, a remaining insulin amount icon, a last bolus infusion information icon and a remaining time icon of the infusion device.

Compared with the prior art, the technical solution of the present invention has the following advantages:
In the drug delivery system with graphical user interface disclosed by the present invention, the graphical user interface comprising a main screen, the main screen comprising an insulin information display area and a blood glucose information display area, the graphical user interface displays the insulin infusion status information in a graphical and/or textual manner, the graphic is a ring with at least one color indicating the type and the progress of the insulin infusion, the text comprising an abbreviation of the type of infusion and the rate or the progress of the infusion, thus the user can intuitively know the insulin infusion status information and blood glucose information at that time and ensure the infusion state.

Furthermore, when the detection device is in the general detection state, the status display area of the detection device displays the blood glucose reading at the latest moment, and displays the blood glucose values in different blood glucose states with different colors. At the same time, arrows in different colors, numbers, and directions indicate the change trend of blood glucose. The blood glucose reading and blood glucose trend arrows are set in the ring indicating the type and progress of insulin infusion, thus users can intuitively know the current insulin infusion status, blood glucose level and blood glucose change trend at the same time.

Furthermore, when the detection device is in a special detection state, the detection device state display area displays text information with specific meaning to remind the user that the detection device or the blood glucose value detected by the detection device is abnormal. The user can timely know the special state of the detection device and take corresponding measures to change the state of the infusion device.

Furthermore, the blood glucose graph display area includes a blood glucose reading curve or discrete points of blood glucose reading for a period of time, as well as the normal blood glucose value area or the standard line of high and low blood glucose values. By comparing the blood glucose reading with the high and low blood glucose values graphically, users can intuitively know the area where the blood glucose value exceeds the normal blood glucose value in the past period of time, and the rough change rate of the blood glucose.

Furthermore, the main screen also includes a detection device calibration display area, which is used to display the next calibration progress of the detection device and remind the user to calibrate the detection device.

Furthermore, the main screen also includes the icon of suspending infusion function to remind the user that the drug infusion system is currently under the protection of hypoglycemia suspending infusion function or predicting hypoglycemia suspending infusion function.

Furthermore, the main screen also includes the blood glucose data recovery icon. When the detection device is reconnected after a loss of connection, it will remind the user that the data is being recovered and will disappear after the recovery, which will not affect the viewing of blood glucose data.

In the drug delivery system with graphical user interface disclosed by the present invention, the user interface includes a main screen, which includes the insulin information display area and the blood glucose information display area. The main screen can slide into other interfaces, and the user can operate independently and easily. The sliding mode can be defined independently, and improving the user experience.

Furthermore, the drug infusion system with graphical user interface can return to the main screen from other interfaces by sliding. The way of returning to the main screen is related to the way of accessing the main screen, which is convenient for users to remember.

Furthermore, the drug infusion system with graphical user interface can also make the user interface return to the main interface from any interface in a unified way, which is simple to operate and improves the user experience.

In the drug infusion system with a graphical user interface disclosed by the present invention, the user interface includes a main screen, the main screen includes an insulin information display area and a blood glucose information display area, the insulin information display area includes insulin infusion status information, and the blood glucose information display area includes a blood glucose graphical display area, which displays the blood glucose level of the user in the first time period in a graphical manner, through a user input, the graphical user interface displays the blood glucose level of the user in other time periods, so that the user can intuitively know the current insulin infusion status information and the blood glucose information in different time periods.

Furthermore, in response to different user inputs, the blood glucose information in different time periods can be displayed on the main screen or the extended blood glucose information display area which is at least partially covered the main screen, so that users can learn about blood glucose information in different time periods in various forms and improve the user experience.

Furthermore, in response to user input, the detailed blood glucose information interface can also be accessed from the blood glucose graphic display area. The detailed blood glucose information interface at least includes basic statistics and/or distribution statistics, so that users can learn more and more specific blood glucose information.

In the drug infusion system with a graphical user interface disclosed by the present invention, the user interface includes a main screen, the main screen includes an insulin infusion status information, and the user interface displays insulin infusion status information in the form of graphics and/or text, the graphic is a ring with at least one color, representing the type and progress of insulin infusion, and the text includes the abbreviation of the infusion type and the infusion rate or infusion progress. Users can clearly know the current insulin infusion type, infusion rate or infusion progress, and confirm the drug infusion status, so that users can intuitively understand the insulin infusion status information.

Furthermore, the main screen also includes a graphic display area of insulin infusion information, which displays insulin infusion information for a period of time in the form of a graph, making it easier for users to intuitively know insulin infusion status information for a period of time in the past.

Furthermore, in response to different user inputs, insulin infusion status information in different time periods can be displayed on the main screen or the expanded insulin infusion status information display area at least partially covering the main screen, so that users can learn about insulin infusion information in different time periods in various forms and improve the user experience.

Furthermore, in response to user input, the detailed insulin infusion information interface can also be accessed from the insulin infusion information display area. The detailed insulin infusion information interface at least includes basic statistical data, so that users can learn more and more specific insulin infusion information.

Furthermore, when the infusion device is in a special state, the insulin infusion state information is displayed as a text information with a specific meaning to remind the user that the infusion device is in a special state. The user can timely know the special state of the infusion device and take corresponding measures to change the state of the detection device.

Furthermore, the home screen at least includes IOB icon, the remaining insulin amount icon, the last bolus infusion information icon and the remaining time icon of the infusion device, so that users can learn more information and ensure the safety of infusion.

### BRIEF DESCRIPTION OF THE DRAWINGS

FIG. 1 is a schematic diagram of the drug delivery system with graphical user interface according to an embodiment of the present invention.
FIG. 2 is a default home screen of the control device according to an embodiment of the present invention.
FIG. 3 is a schematic diagram of the main screen of the drug infusion system with a graphical user interface when the blood glucose value is a discrete value according to an embodiment of the present invention.
FIG. 4 is a schematic diagram of the main screen of the drug infusion system with a graphical user interface when the blood glucose data is in recovery state according to an embodiment of the present invention.
FIG. 5 is a schematic diagram of the main screen of the drug infusion system with a graphical user interface when the hypoglycemic suspend infusion function or the predicted hypoglycemic suspend infusion function is turn on according to an embodiment of the present invention.
FIG. 6 is a schematic diagram of an expanded blood glucose graphical display area of the drug infusion system with a graphical user interface according to an embodiment of the present invention.
FIG. 7 is a schematic diagram of the main screen of the drug infusion system with a graphical user interface when an alert event occurs according to an embodiment of the present invention.
FIG. 8 is a schematic diagram of the main menu interface of the drug infusion system with a graphical user interface according to an embodiment of the present invention.
FIG. 9 is a schematic diagram of a status interface of the drug infusion system with a graphical user interface according to an embodiment of the present invention.
FIG. 10 is a schematic diagram of an alert notification interface of the drug infusion system with a graphical user interface according to an embodiment of the present invention.
FIG. 11 is a schematic diagram of a shortcut interface of the drug infusion system with a graphical user interface according to an embodiment of the present invention.
FIG. 12 is a schematic diagram of an event icon of the drug infusion system with a graphical user interface according to an embodiment of the present invention.
FIG. 13 is a schematic diagram of a detailed blood glucose information interface of the drug infusion system with a graphical user interface according to an embodiment of the present invention.
FIG. 14 is a schematic diagram of another main screen of the drug infusion system with a graphical user interface of according to another embodiment of the present invention.
FIG. 15 is a schematic diagram of a default home screen of the drug infusion system for displaying drug infusion information according to an embodiment of the present invention.
FIG. 16 is a schematic diagram of an expanded insulin infusion information graphic display area of the drug infusion system for displaying drug infusion information according to an embodiment of the present invention.
FIG. 17 is a schematic diagram of a detailed insulin infusion information interface of the drug infusion system for displaying drug infusion information according to an embodiment of the present invention
FIG. 18 is a schematic diagram of another main screen of the drug infusion system for displaying drug infusion information according to an embodiment of the present invention.

### DETAILED DESCRIPTION

As mentioned above, in the prior art, the user can not intuitively know the insulin infusion status information and/or blood glucose information they need to pay attention to, and cannot operate it autonomously and conveniently, resulting in a poor user experience.

In order to solve this problem, the present invention provides a drug delivery system with graphical user interface, the control device provides a graphical user interface, and the graphical user interface includes a main screen, and the main screen displays insulin infusion status information and blood glucose information, and the graphical user interface displays the insulin infusion status information in a graphical and/or textual manner to facilitate the user to intuitively know the insulin infusion status information and blood glucose information at that time.

Various exemplary embodiments of the present invention will now be described in detail regarding the figures. The relative arrangement of the components and the steps, numerical expressions and numerical values outlined in the embodiments are not construed as limiting the scope of the invention.

In addition, it should be understood that, for ease of description, the dimensions of the various components shown in the figures are not necessarily drawn in the actual scale relationship; for example, the thickness, width, length or distance of certain units may be exaggerated relative to other mechanism modules.

The following description of the exemplary embodiments is merely illustrative and does not limit the invention its application or use. The techniques, methods, and devices are known to those of ordinary skill in the art and may not be discussed in detail. However, such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

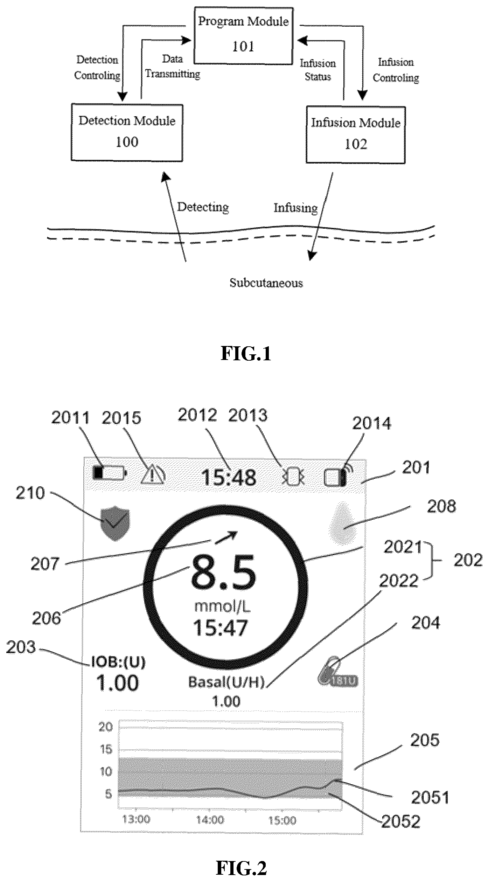

FIG. 1 is a schematic diagram of the drug delivery system with graphical user interface according to an embodiment of the present invention.

The drug infusion system with graphical user interface disclosed in the embodiment of the present invention mainly includes a detection device 100, a control device 101 and an infusion device 102.

The detection device 100 is used to continuously detect the current blood glucose value of the user. Generally, the detection device 100 is Continuous Glucose Monitoring (CGM), which can detect the current blood glucose value of the user in real time, monitor the blood glucose change, and send the current blood glucose value to the control device 101.

The control device 101 is used to control the operation of the detection device 100 and the infusion device 102. The control device 101 is a portable electronic device, such as a smart phone, PDM, smart watch, handheld, etc. The portable electronic device may include: a communication interface for communicating with the detection device, the infusion device and the external remote device, etc.; a display and a display controller, which may present visual information in graphics and/or text manner and control the presentation of visual information; an input device, such as a mouse, a keyboard, touch screen, microphone, etc., for receiving input of signals; memory and processor, etc., memory for storing data, records, instructions, etc., and processor for executing instructions in memory, controlling the operation of other components, etc.

The infusion device 102 contains all the mechanical components necessary to infuse insulin and is controlled by the control device 101, such as an insulin pump. According to the insulin infusion instruction from the control device 101, the infusion device 102 deliveries the currently required insulin into the user's body. At the same time, the infusion status of the infusion device 102 can be fed back to the control device 101 in real time.

In one embodiment of the present invention, the detection device 100 and the infusion device 102 are provided in separate devices. Therefore, they are attached to different positions of the user's skin respectively. At this time, the control device 101, the detection device 100 and the infusion device 102 transmit wireless signals to each other for connection.

In another embodiment of the present invention, the detection device 100 and the infusion device 102 are provided in the same device, such as the detection device 100 is integrated in the infusion device 102, and the control device 101 directly transmits wireless signals with the detection device 100 and the infusion device 102 for connection.

FIG. 2 is a default home screen of the control device according to an embodiment of the present invention.

In this embodiment of the present invention, the main screen includes a status bar icon area 201, and the status bar icon area 201 includes a battery icon 2011 for displaying the remaining battery status of the control device 101 or the battery status when charging; a time icon 2012 for displaying the current time; an audio icon 2013 for indicating different reminders, such as audio, vibration, or audio + vibration; a communication icon 2014, communicating with the infusion device 102, for displaying communication connection the status with the infusion device 102; alert icon 2015, for displaying alerts of different priority levels.

The main screen also displays insulin information, specifically including the insulin delivery status icon 202, insulin on board, IOB icon 203, and the remaining insulin amount icon 204 in the infusion device 102, wherein the insulin delivery status icon 202 includes a graphic 2021 and/or text 2022, wherein the graphic 2021 is a ring of different colors, indicating different infusion types, and expressing the progress of infusion with a change in color, and text 2022 showing the abbreviation of the insulin infusion type and the infusion rate or progress of infusion. When the ring 2021 is gray, it indicates that the infusion device 102 is not activated; when the ring 2021 is green, it indicates that the basal infusion is in progress; when the ring 2021 is a green ring with dark green, it indicates that the temp basal infusion is in progress and the dark green part indicates the progress of the temp basal infusion; when the ring 2021 is a blue ring with dark blue, the blue ring indicates a normal bolus infusion, and the dark blue portion indicates infusion progress; when the ring 2021 is a purple ring with dark purple, the purple ring indicates an extended bolus infusion, and the dark purple portion indicates infusion progress; when the ring 2021 is red, it indicates infusion suspension. Text section 2222 Basal(U/H) 1.00 indicates the current basal infusion rate is 1.00 U/H; Temp Basal(U/H) 1.00: indicates temp basal infusion activated and temp basal rate is 1.00 U/H; Temp Basal(U/H) 1.00 85%: indicates temp basal activated, temp basal rate is 1.00 U/H and 85% of current basal rate; Normal(U) 1.00/2.00 : indicates that the normal bolus activated, 1U normal bolus infusion delivered, and the total amount of normal bolus is 2U; Extended(U) 1.00/2.00: indicates that the extended bolus activated, 1U of extended bolus infusion delivered, and the total amount of extended bolus is 2U; C-Normal(U) 1.00/2.00: normal bolus of combo bolus activated, and 1U normal bolus infusion delivered, the total amount of normal bolus in the combo bolus is 2U; C-Ext.(U) 1.00/2.00: the extended bolus of combo bolus activated, and 1U extended bolus infusion delivered, the total amount of normal bolus in the combo bolus is 2U; Suspend time remaining 0:15 indicates that the suspension has been activated and basal will resume automatically after 15 minutes. In the embodiment of the present invention, the graph 2021 and the text 2022 cooperate to jointly display the current insulin infusion status, and the user can intuitively know the current insulin infusion type, infusion rate or infusion progress and confirm the drug infusion status through both the graph and the text. In other embodiments of the present invention, the main screen can also display the current insulin infusion status only through graphics 2021 or text 2022 alone, and the user can also intuitively know the current insulin infusion type, infusion rate or infusion progress.

The main screen also displays blood glucose information, and the blood glucose information includes the blood glucose graphic display area 205 and the status display area 206 of the detection device 100, when the detection device 100 is in the general detection state, the display area 206 shows the blood glucose reading of the latest moment, when the blood glucose value is in the normal blood glucose value range, the blood glucose reading is shown in green, when the blood glucose value is lower than the low blood glucose standard value, the blood glucose reading is shown in yellow; when the blood glucose value is higher than the high blood glucose standard value, the blood glucose reading is shown in red. When the detection device 100 is in the special detection state, the display area 206 shows other text information, such as, Warm-Up, which means the detection device 100 is warming up, reminding the user to wait for a while; ERR, which means the detection device 100 needs to be calibrated after 15 minutes; BG-, which means the detection device 100 needs to be calibrated immediately; ????? indicates that the detection device 100 has no reading, reminding the user to wait or further check whether the detection device 100 needs to be replaced; LOST, indicates that the signal of the detection device 100 has been lost for at least 10 minutes, reminding the user to reconnect the detection device 100; High, indicates that the blood glucose value detected by the detection device 100 is higher than a set threshold, and the threshold value seriously exceeds the generally recognized high blood glucose standard. For example, the generally recognized high blood glucose standard value is 13.3 mmol/L (400mg/dL), while the threshold value is 22.2 mmol/L (400mg/dL), reminding the user to take measures to change the blood glucose status; Low, indicating that the blood glucose value detected by the detection device 100 is lower than a set threshold value, and the threshold value is seriously lower than the generally recognized low blood glucose standard, for example, the generally recognized low blood glucose standard is 4.4 mmol/L (400mg/dL), while the threshold value is set to 2.2 mmol/L (40 mg/dL), reminding the user to take measures to change the blood glucose status; When the display is an underlined blood glucose reading, it indicates that the calibration of the detection device 100 has expired at that monitoring moment, reminding the user that the blood glucose value at that moment may be inaccurate. The display area 206 of the detection device 100 is provided inside the ring 2021.

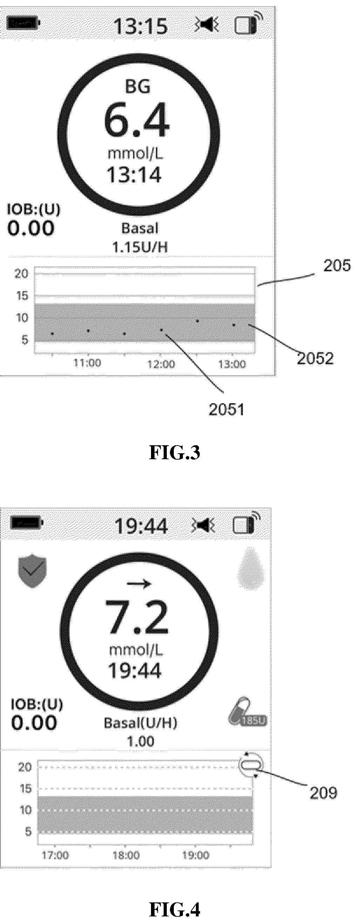

The blood glucose graphical display area 205 includes the blood glucose reading curve or blood glucose reading discrete points 2051 over a period of time, and the normal blood glucose value area 2052, which is convenient for the user to intuitively know the blood glucose level in the past and current time period, as shown in FIG. 2 and FIG.3, the normal blood glucose value area 2052 is an area filled with certain colors; in another embodiment of the present invention, it is also possible to display the normal blood glucose value area only by highlighting and/or bolded the standard line of high and low blood glucose value, the user can also intuitively know the blood glucose level in the past and current time period as well as the rough blood glucose change rate. By user input, such as touching and holding or sliding the graphic display area 205, a cursor will appear and when the cursor is moved to a point on the blood glucose reading curve, the detection time and reading of the blood glucose value will be displayed near that point, and the reading can also be displayed in different colors, such as green when the reading is within the preset range, and red when the reading is outside the preset range. When the cursor moves to a certain point, at that moment the detection device is in the special detection state as described before, the text information such as "Warm-Up", "BG-", etc. will also be displayed near the point, and the meaning of each text is the same as described before. The blood glucose value information or special detection status information is displayed near this point for a certain period of time, such as 1s, 2s, or 5s, and then disappears.

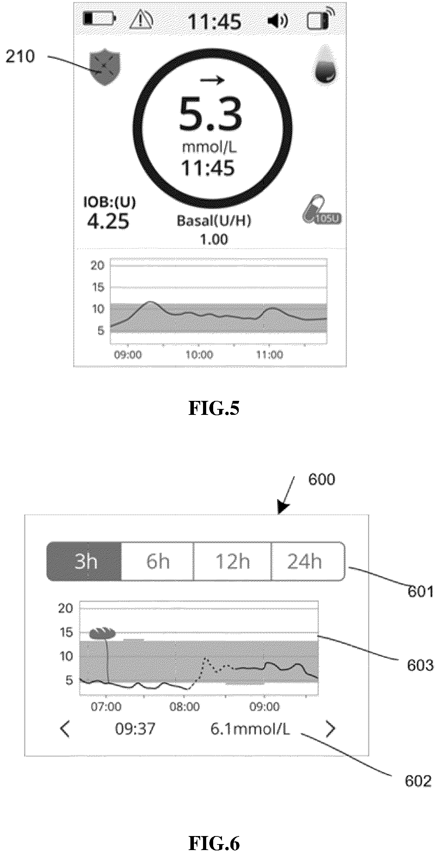

In one embodiment of the present invention, by user input, such as long pressing the blood glucose graphic display area 205, the blood glucose graphic display area 205 can be rotated to rotate horizontally and enlarge the graphic display area, and the content of the graphic display area can be expanded to an expanded blood glucose graphic display area 600, as shown in FIG. 6. The expanded blood glucose graphic display area 600 can partially cover the main screen or fully cover the main screen area. Preferably, the expanded blood glucose graphic display area 600 is fully covered the main screen area, so that the expanded blood glucose graphical display area 600 can display more content clearly for the user to know more information. The expanded graphical display area 600 includes a time period selection area 601, a blood glucose text display area 602 and a blood glucose graphical display area 603, the blood glucose text display area 602 is the status display area of the detection device, and the blood glucose level in the corresponding different time periods can be viewed by clicking different time periods in the time period selection area 601, and the time period displayed on the horizontal axis of the blood glucose graphical display area 603 is the same as the time period selected in the time period selection area 601. By user input, such as touching and holding or sliding the blood glucose graphic display area 603, a cursor will appear, and when moving the cursor to a point on the blood glucose reading curve, the blood glucose text display area 602 will display the detection time and reading of the blood glucose value, and the reading can also be displayed in different colors, such as green when the reading is within a preset range, and can be displayed in red when the reading is outside the preset range. Click the arrows on both sides of the blood glucose value text display area 602 can select different time periods, and the blood glucose graph display area 205 displays the blood glucose reading curve or the discrete points of the blood glucose reading within the corresponding time period, and when moving the cursor to a certain point on the blood glucose reading curve, the blood glucose text display area 602 will display the detection time and reading of the blood glucose value, and the reading can also be displayed in different colors, such as green when the reading is within the preset range, and red when the reading is outside the preset range. When the cursor moves to a certain point, when the detection device is in the special detection state as described above, the blood glucose value text display area 602 will also display the text information such as "Warm-Up" and "BG-" as described above, and the meaning of each text is also the same as described above.

In another embodiment of the present invention, the expanded blood glucose graphic display area 600 may also not include the blood glucose text display area 602, when moving the cursor to a point on the blood glucose reading curve, the blood glucose value and detection time or special detection status at that moment are displayed near that point, and disappear after displaying for a certain time, such as 1s, 2s, 5s, and the display mode of the blood glucose value and detection time, such as color, the text meaning of the special detection status is the same as described before, and will not be repeated here.

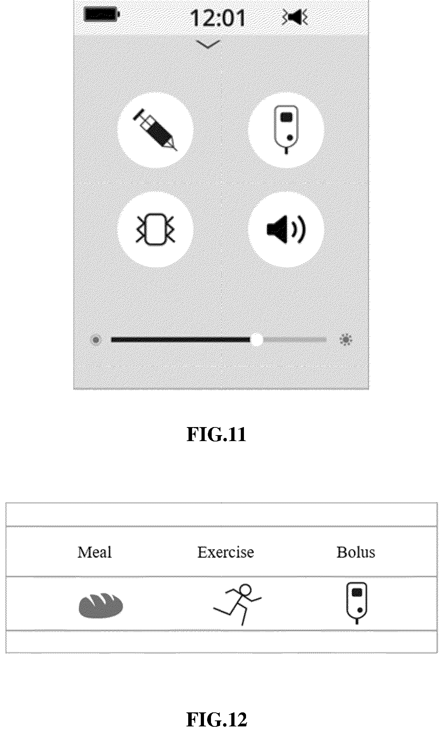

When a certain event occurs, such as meal, including breakfast, lunch, dinner and snack, exercise, and bolus infusion, the corresponding event indication icon, as shown in FIG.12, will be displayed in the blood glucose graphic display area 603, shown as 604, for indicating the time when the specific event occurs. The user can intuitively know the events such as meal, exercise, etc. that occurred during the specified time period. Since the detection device 100 is continuously detecting blood glucose value, the blood glucose reading curve on the blood glucose graphic display area 603 will be continuously updated, and the event indication icon 604 will be moved with the time update on the horizontal axis of the blood glucose graphic display area 603, and the event indication icon will disappear when the time of the event occurred beyond the time period shown on the horizontal axis of the blood glucose graphic display area 603.

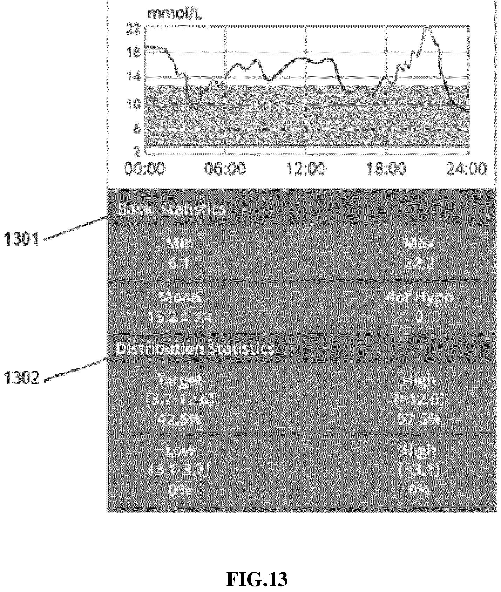

By user input, such as clicking or double-clicking the blood glucose graphic display area 603 will enter into the detailed blood glucose information interface, as shown in FIG.13, the detailed blood glucose information interface includes the basic statistics display area 1301 and the distribution statistics display area 1302, the basic statistics display area 1301 includes the minimum, maximum and average values of the detected blood glucose, and the number of hypoglycemic episodes (below 3.1 mmol/L / 56mg/dL). The distribution statistics display area 1302 includes the percentage of blood glucose values within the target blood glucose range and the percentage of blood glucose values above the target blood glucose range. The percentage of blood glucose values within the set hypoglycemic range and the percentage of blood glucose values below the set hypoglycemic range (below 3.1 mmol/L /56 mg/dL).

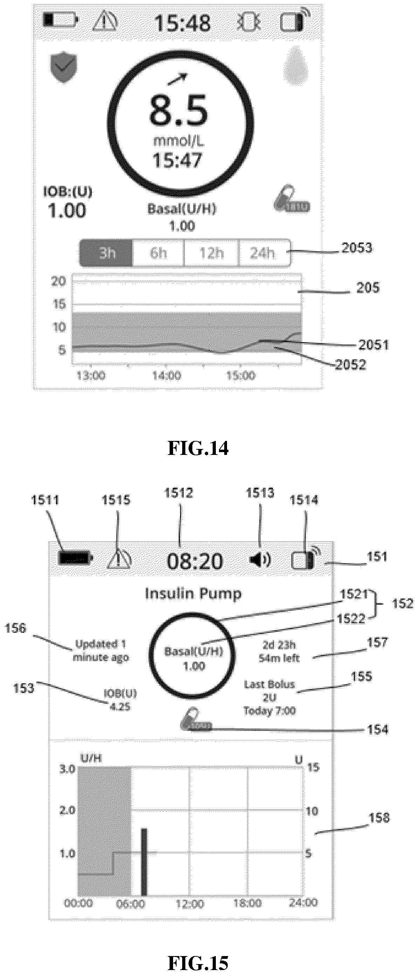

In another embodiment of the present invention, by user input, such as clicking on the blood glucose display area 205, a time period selection area 2053 will be displayed on the main screen, as shown in FIG. 14, and different time periods can be selected to view the blood glucose levels during the corresponding different time periods, and the time period displayed on the horizontal axis of the blood glucose graphic display area 205 is the same as the time period selected in the time period selection area 2053. By user input, such as touching and holding or sliding the graphical display area 205, a cursor will appear, and when moving the cursor to a point on the blood glucose reading curve, the detection time and the blood glucose reading will be displayed near that point, and the reading can also be displayed in different colors, such as green when the reading is within the preset range, and can be displayed in red when the reading is outside the preset range. Similarly, by user input, such as double-clicking on the blood glucose graphical display area 205 will enter the detailed blood glucose information interface as shown in FIG. 13.

When the detection device 100 is in the general detection state, the blood glucose information also includes the blood glucose trend display area 207. The blood glucose trend display area 207 is indicated by indicator arrows with certain color, direction and number, to show the change trend and speed of the blood glucose reading with different color, direction and number, such as a green arrow to the right indicates that the blood glucose status is stable and tells the user that no special attention to the blood glucose is needed at present; an orange arrow to the upper right indicates that the blood glucose is slowly rising; an orange arrow to the lower right indicates that blood glucose is slowly falling, reminding the user to pay attention to further changes in blood glucose; an upward red arrow indicates that blood glucose is rising; a downward red arrow indicates that blood glucose is falling; two upward red arrows indicate that blood glucose is rising rapidly, reminding the user that he or she needs to pay attention to changes in blood glucose at all times or to start adjusting current insulin infusion strategy; two downward red cut-offs indicate a rapid drop in blood glucose, reminding the user that immediate action is needed to change the current blood glucose status, such as carbohydrate supplementation, bolus infusion, etc. Both blood glucose reading 206 and blood glucose trend indication arrow 207 are set inside the ring 2021, indicating the type and progress of insulin infusion, and the user can intuitively know the current insulin infusion status and blood glucose value and blood glucose trend at the same time.

The main screen also includes the calibration display area 208 of the detection device 100. The calibration display area 208 dynamically displays the calibration status of the detection device100 over time. In this embodiment of the invention, the detection device 100 is CGM, and when the CGM function is turned on, the calibration display area 208 is a teardrop-shaped icon and indicates how long it will take for the next calibration to be completed with a different amount of teardrop color filling, as shown in the calibration display area 208 in FIG.2, the next calibration will be completed in 4-6 hours, i.e. the user is going to complete the CGM calibration after 4-6. In another embodiment of the present invention, the detection device is 100 non-CGM, such as a fingertip blood collector, when the detection device 100 does not require frequent calibration, so there is no calibration display area 208 and the blood glucose graphical display area 205 shows discrete blood glucose value points, as shown in FIG. 3.

The main screen may also include a blood glucose data recovery icon 209, when the detection device 100 has been reconnected after a loss of connection, the blood glucose data takes some time to recover, therefore, during the period of the blood glucose data recovery, the blood glucose data recovery icon 209 appears in the blood glucose graphic display area 205, as shown in FIG. 4, and when the blood glucose data is fully recovered, the blood glucose data recovery icon 209 disappears.

The main screen may also include the suspend infusion function icon 210, when the hypoglycemia suspend infusion function or the predicted hypoglycemia suspend infusion function in the drug infusion system is turned on, i.e., when the sensor reading of the detection device 100 triggers the suspend function, the drug infusion device 102 will perform a safety detection and automatically suspend the infusion, and the suspend infusion function icon 210 will be displayed on the main screen, as shown in FIG. 2. At that time, the suspend infusion function icon 210 resembles a bright yellow shield and there is a " √ " sign in the shield to remind the user that the drug infusion system is currently providing the safety protection function; when the hypoglycemic suspend infusion function or the predicted hypoglycemic suspend infusion function is off or temporarily unavailable, the suspend infusion function icon 210 on the main screen resembles a gray shield, and there is an "x" sign in the shield, as shown in FIG.5, reminding users that the system is temporarily unable to provide the hypoglycemic suspend infusion function or predicted hypoglycemic suspend infusion function, need to wait for a while for the hypoglycemic suspend infusion function or predicted hypoglycemic suspend infusion function to resume, or to turn on the hypoglycemic suspend infusion function or predicted hypoglycemic suspend infusion function. In another embodiment of the present invention, the suspend infusion function icon 210 may also not be displayed on the main screen when the hypoglycemia suspend infusion function or the predicted hypoglycemia suspend infusion function is turned off or temporarily unavailable.

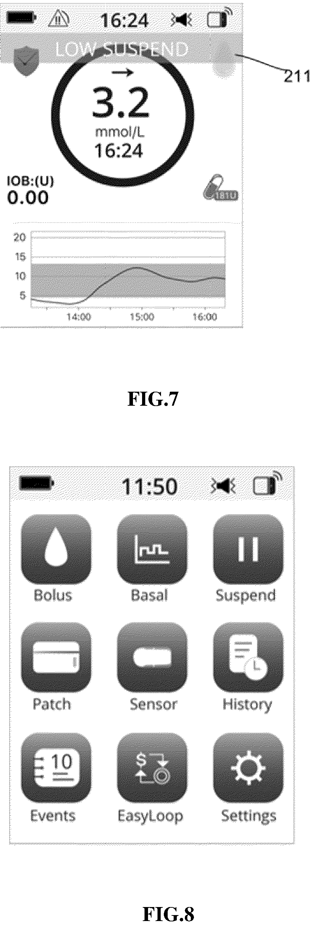

The main screen may also include an alert status icon 211, in one embodiment of the present invention, even though some alerts have been cleared manually, but the alert event has not been resolved, the alert status icon 211 will still be displayed on the main screen, as shown in FIG. 7, such as the infusion device 100 battery is depleted; the drug amount in the infusion device 100 is 0, i.e., the user forgets to fill the drug into the infusion device; the infusion amount exceeds the total daily insulin amount; infusion is suspended because of hypoglycemia, infusion is suspended because of predicted hypoglycemia, etc., the user is prompted that the alert event has not been resolved and the user is reminded to resolve the alert event.

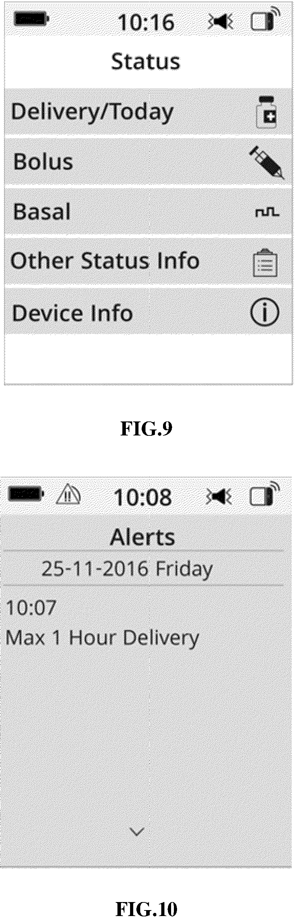

In the embodiment of the present invention, other interfaces can also be accessed by sliding on the main screen, specifically, sliding once in different directions to access different interfaces, such as sliding once from right to left to access the main menu interface, as shown in FIG. 8; sliding once from left to right to access the status interface, as shown in FIG. 9; sliding once from up to down to access the alert notification interface, as shown in FIG. 10; sliding once from up to down to access the shortcut interface, as shown in FIG. 11. There is no restriction on the specific sliding method in this embodiment of the invention, such as in different directions, up and down, left and right, diagonal, and/or number of slides, and the specific interface that can be accessed, which can be set according to user preferences. Likewise, it is possible to return to the home screen from other interfaces by sliding, and the specific sliding return method is not specifically limited here and can be set according to user preferences. Preferably, the way of returning to the home screen is related to the way of entering the home screen, such as sliding in the opposite direction and sliding the same number of times, which is easy for the user to remember. For example, sliding once from left to right can return to the home screen from the main menu interface, sliding once from right to left can return to the home screen from the status interface, sliding once from down to up can return to the home screen from the alert notification interface, and sliding once from up to down can return to the home screen from the shortcut interface. Similarly, the sliding method with same sliding direction but different sliding number can be used to return to the home screen.

As shown in FIG. 8, the main menu interface can include several sub-menus, such as bolus, basal, patch, sensor, history, events, settings, etc. The specific sub-menu categories, number, layout in the main menu interface, and icons of each sub-menu can be set according to the user's preference, and there is no specific restrictions here. Click each sub-menu interface to access different sub-menu interface.

As shown in FIG. 9, the status interface can include multiple status information, such as infusion information, bolus information, basal information, infusion device 102 information, other status information, etc. The specific status information category, quantity, the layout of the status interface, and the icon of each status information can be set according to the user's preference, and no specific restrictions are made here. Clicking on each status information can enter a different status information interface. In the embodiment of the present invention, the alert notification interface only displays the alert notifications that are still valid within the current usage date, as shown in FIG. 10, the alert notifications include the time of alert occurrence and the content of the alert, the alert notifications can be arranged in the order of alert occurrence, such as the latest notification is displayed at the top, the content of the alert notifications and the layout of the alert notification interface can also be arranged according to the user's preference, no specific restrictions are made here.

As shown in FIG. 11, the shortcut interface includes blood glucose shortcut icon, calibration shortcut icon, bolus shortcut icon, audio selection shortcut icon, vibration selection shortcut icon, brightness adjustment shortcut icon, etc. The specific categories, number, layout and the specific icon of each shortcut icon can be set according to the user's preference, no specific restrictions are made here. Clicking each shortcut icon can access different shortcut interfaces.

In an embodiment of the present invention, the user interface can also be set in a particular way to return to the main screen from any interface, for example, in an embodiment of the present invention, the user interface is set with a touch-sensitive button, when the user interface is in any interface other than the main screen, the user can return to the main screen by touching the touch-sensitive button or long press the touch-sensitive button, generally, the touch-sensitive button is set in the lower part of the user interface, or it can be set in any part of the user interface depending on the user's preference. In another embodiment of the present invention, tapping the user interface twice or more times in a row returns to the home screen. In yet another embodiment of the present invention, returning to the home screen in a specific sliding direction and number of times, such as sliding the user interface twice or more times in succession in any direction to return the user interface to the home screen from any interface, such as sliding from the center of the user interface to any two or more directions at the same time to return the user interface to the home screen from any interface with two or more fingers, or sliding from any direction to the center of the user interface at the same time to return the user interface to the home screen from any interface with two or more fingers, or the method for returning the user interface to the home screen can be defined only by a single sliding in a single direction, such as a single sliding in a horizontal direction, a vertical direction, or a diagonal direction.

In another embodiment of the present invention, the control device further comprises a solid button, the user interface can be returned to the home screen from any interface by pressing the solid button.

In another embodiment of the present invention, when the detection device 100 is not enabled, the control device 101 only controls the operation of the infusion device 102, and the user interface of the control device 101 only displays information related to insulin infusion. The main screen of the control device 101 when the detection device 100 is not enabled is shown in FIG. 15. The main screen includes a status bar icon area 151, and the status bar icon area 151 includes a battery icon 1511 for displaying the remaining battery status of the control device 101 or the battery status while charging; a time icon 1512 for displaying the current time; an audio icon 1513 for indicating different reminders, such as audio, vibration, or audio+vibration; a communication icon 1514, communicating with the infusion device 102; and alert icon 1515 for displaying alerts of different priority levels.

The main screen also displays insulin information, specifically including the insulin delivery status icon 152, insulin on board, IOB icon 153, the remaining insulin amount icon 154 in the infusion device 102, the last bolus infusion information icon 155, the last bolus infusion information including the amount and time of the last bolus infusion, the insulin infusion information update icon 156 and the remaining time icon 157 of the infusion device 102. It is noted that in this embodiment of the invention, the infusion device 102 includes a reusable pump controller and a single-use patch, so the remaining time icon 157 of the infusion device 102 indicates the remaining time that the single-use patch needs to be replaced.

The insulin delivery status icon 152 includes a graphic 1521 and/or text 1522, wherein the graphic 1521 is a ring of different colors, indicating different infusion types, and expressing the progress of infusion with a change in color, and text 1522 showing the abbreviation of the insulin infusion type and the infusion rate or progress of infusion. When the ring 1521 is gray, it indicates that the infusion device 102 is not activated; when the ring 1521 is green, it indicates that the basal infusion is in progress; when the ring 1521 is a green ring with dark green, it indicates that the temp basal infusion is in progress and the dark green part indicates the progress of the temp basal infusion; when the ring 1521 is a blue ring with dark blue, the blue ring indicates a normal bolus infusion, and the dark blue portion indicates infusion progress; when the ring 1521 is a purple ring with dark purple, the purple ring indicates an extended bolus infusion, and the dark purple portion indicates infusion progress; when the ring 1521 is red, it indicates infusion suspension. Text section 1522 Basal(U/H) 1.00 indicates the current basal infusion rate is 1.00 U/H; Temp Basal(U/H) 1.00: indicates temp basal infusion activated and temp basal rate is 1.00 U/H; Temp Basal(U/H) 1.00 85%: indicates temp basal activated, temp basal rate is 1.00 U/H and 85% of current basal rate; Normal(U) 1.00/2.00 : indicates that the normal bolus activated, 1U normal bolus infusion delivered, and the total amount of normal bolus is 2U; Extended(U) 1.00/2.00: indicates that the extended bolus activated, 1U of extended bolus infusion delivered, and the total amount of extended bolus is 2U; C-Normal(U) 1.00/2.00: normal bolus of combo bolus activated, and 1U normal bolus infusion delivered, the total amount of normal bolus in the combo bolus is 2U; C-Ext.(U) 1.00/2.00: the extended bolus of combo bolus activated, and 1U extended bolus infusion delivered, the total amount of normal bolus in the combo bolus is 2U; Suspend time remaining 0:15 indicates that the suspension has been activated and basal will resume automatically after 15 minutes. In the embodiment of the present invention, the graph 1521 and the text 1522 cooperate to jointly display the current insulin infusion status, and the user can intuitively know the current insulin infusion type, infusion rate or infusion progress and confirm the drug infusion status through both the graph and the text. Preferably, this text 1522 is set in the graphics 1521 to make the layout of the user interface more reasonable and aesthetically, and further enhance the user experience. In other embodiments of the present invention, the main screen can also display the current insulin infusion status only through graphics 1521 or text 1522 alone, and the user can also intuitively know the current insulin infusion type, infusion rate or infusion progress. It should be noted that the specific meaning represented by the shape and color of the graphics, as well as the specific meaning represented by the different texts are not limit here, which can be customized by the user according to personal habits or preferences.

When the infusion device 102 is in a special state, the insulin delivery status icon 152 will be replaced with other alert text to remind the user of the abnormality of the infusion device 102, so that the user can understand the special state of the infusion device and take corresponding measures to change the state of the infusion device in time. For example, "pump lost" indicates that the control device 101 has lost the signal of the infusion device 102; "blockage detected" indicates that the infusion device 102 is blocked; "empty reservoir" indicates that no insulin left in the reservoir; "patch expired" means the patch has expired; "patch battery exhausted" means the battery of the patch has been exhausted; "pump controller error" means the pump controller is not working properly.

The main screen also includes an insulin infusion information graphic display area 158, and the horizontal axis of the infusion information graphic display area 158 represents time, the vertical axis on one side represents the infusion rate of basal, and the vertical axis on the other side represents the infusion amount of bolus, displaying the insulin infusion information in a graphical form over a period of time. By user input, such as touching and holding or sliding the graphic display area 158, a cursor will appear, and when moving the cursor to a point on the curve, the amount and time of insulin infusion at that time will be displayed near that point and disappear after displaying a certain time, such as 1s, 2s, or 5s, etc.

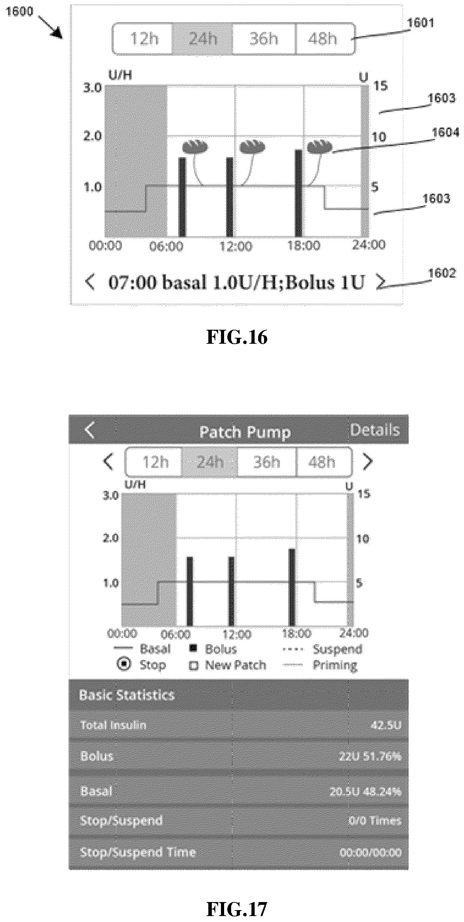

In one embodiment of the present invention, by user input, such as long pressing the insulin infusion information graphic display area 158, the insulin infusion information graphic display area 158 can be rotated to rotate horizontally and enlarge the graphic display area, and the content of the graphic display area can be expanded to an expanded insulin infusion information graphic display area 1600, as shown in FIG. 16. The expanded insulin infusion information graphic display area 1600 can partially cover the main screen or fully cover the main screen area. Preferably, the expanded insulin infusion information graphic display area 600 is fully covered the main screen area, so that the expanded insulin infusion information graphical display area 600 can display more content clearly for the user to know more information. The expanded graphical display area 1600 includes a time period selection area 1601, an insulin infusion information text display area 1602 and an insulin infusion information graphical display area 603, the insulin infusion information text display area 1602 is the status display area of the infusion device, and the insulin infusion information in the corresponding different time periods can be viewed by clicking different time periods in the time period selection area 1601, and the time period displayed on the horizontal axis of the insulin infusion information graphical display area 603 is the same as the time period selected in the time period selection area 1601. By user input, such as touching and holding or sliding the insulin infusion information graphic display area 603, a cursor will appear, and when moving the cursor to a point on the insulin infusion information curve, the insulin infusion information text display area 1602 will display the infusion amount and time. When the cursor moves to a certain point, when the infusion device is in the special detection state as described above, the insulin infusion information text display area 1602 will also display the text information such as "pump lost" and "blockage detected " as described above, and the meaning of each text is also the same as described above.

In another embodiment of the present invention, the expanded insulin infusion information graphic display area 1600 may also not include the insulin infusion information text display area 1602, when moving the cursor to a point on the curve, the insulin infusion information amount and time at that moment are displayed near that point, and disappear after displaying for a certain time, such as 1s, 2s, 5s, etc..

When a certain event occurs, such as meal, including breakfast, lunch, dinner and snack, exercise, the corresponding event indication icon will be displayed in the insulin infusion information graphic display area 1603, shown as 1604, for indicating the time when the specific event occurs. The user can intuitively know the events such as meal, exercise, etc. that occurred during the specified time period. Since the infusion device 102 is continuously infusing insulin, the insulin infusion information curve on the insulin infusion information graphic display area 1603 will be continuously updated, and the event indication icon 1604 will be moved with the time update on the horizontal axis of the insulin infusion information graphic display area 1603, and the event indication icon will disappear when the time of the event occurred beyond the time period shown on the horizontal axis of the insulin infusion information graphic display area 1603.

By user input, such as clicking or double-clicking the insulin infusion information graphic display area 1603 will enter into the detailed insulin infusion information interface, as shown in FIG.17, the detailed insulin infusion information includes the basic statistics, such as, the total insulin infused during the current interface time period, the total amount of bolus and the percentage of the bolus amount to the total amount infused, the total amount of basal and the percentage of the basal to the total amount infused, the number of stops or/suspends, and the stop/suspend time.

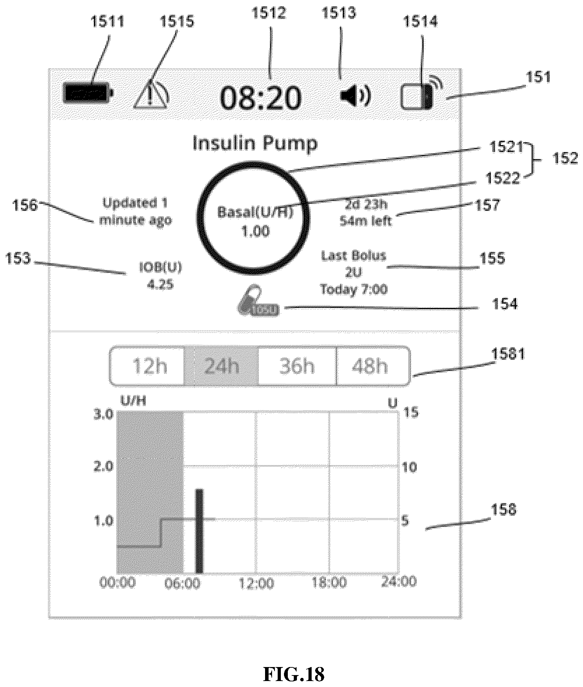

In another embodiment of the present invention, by user input, such as clicking on the insulin infusion display area 158, a time period selection area 1581 will be displayed on the main screen, as shown in FIG. 18, and different time periods can be selected to view the insulin infusion during the corresponding different time periods, and the time period displayed on the horizontal axis of the insulin infusion graphic display area 158 is the same as the time period selected in the time period selection area 1581. By user input, such as touching and holding or sliding the graphical display area 158, a cursor will appear, and when moving the cursor to a point on the insulin infusion curve, the infusion time and amount will be displayed near that point. Similarly, by user input, such as double-clicking on the insulin infusion graphical display area 158 will enter the detailed insulin infusion interface, as shown in FIG. 17.

As a summary, the present invention discloses a drug delivery system with graphical user interface, comprising a detection device, an infusion device and a control device, the control device provides a graphical user interface, and the graphical user interface includes a main screen, and the main screen displays insulin infusion status information and/or blood glucose information, and the graphical user interface displays the insulin infusion status information in a graphical and/or textual manner to facilitate the user to intuitively know the insulin infusion status information.

While the invention has been described in detail regarding the specific embodiments of the present invention, it should be understood that it will be appreciated by those skilled in the art that the above embodiments may be modified without departing from the scope and spirit of the invention. The appended claims define the scope of the invention.

## Claims

1. A drug delivery system with graphical user interface, comprising:
a detection device, for detecting the blood glucose level of the user;
an infusion device, for infusing a drug into the user's body; and
a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein the insulin information display area comprising insulin infusion status information, the graphical user interface displays the insulin infusion status information in a graphic and/or text manner.

2. The drug delivery system with graphical user interface of claim 1, wherein
the graphic is a ring with at least one color indicating a type and progress of the insulin infusion, the text comprising an abbreviation of a type of infusion and rate or progress of the infusion.

3. The drug delivery system with graphical user interface of claim 2, wherein
the blood glucose information includes a blood glucose graphic display area and a status display area of the detection device.

4. The drug delivery system with graphical user interface of claim 3 wherein
the status display area of the detection device area is set inside the ring.

5. The drug delivery system with graphical user interface of claim 4, wherein
the detection device status display area displays information that the detection device is in a general detection state or a special detection state.

6. The drug delivery system with graphical user interface of claim 5, wherein
when the detection device is in the general detection state, the detection device status display area shows the blood glucose reading of the latest moment.

7. The drug delivery system with graphical user interface of claim 5, wherein
when the detection device is in the special detection state, the detection device status display area shows a text message with a specific meaning to alert the user that an abnormal blood glucose value was detected by the detection device or there is an abnormal in the detection device.

8. The drug delivery system with graphical user interface of claim 3, wherein
the blood glucose graphical display area includes a blood glucose reading curve or blood glucose reading discrete points over a period of time, and the normal blood glucose value area or standardized lines for high blood glucose values and low blood glucose values.

9. The drug delivery system with graphical user interface of claim 6, wherein
when the detection device is in the general detection state, the detection device status display area further includes a blood glucose trend display area.

10. The drug delivery system with graphical user interface of claim 9, wherein
the blood glucose trend display area is indicated by indicator arrows with different color, direction and number.

11. The drug delivery system with graphical user interface of claim 10, wherein
the blood glucose trend display area is set in the ring.

12. The drug delivery system with graphical user interface of claim 1, wherein
the main screen further includes a calibration display area of the detection device.

13. The drug delivery system with graphical user interface of claim 12, wherein
the calibration display area dynamically displays the calibration status of the detection device over time.

14. The drug delivery system with graphical user interface of claim 1, wherein
the main screen further includes a blood glucose data recovery icon, the blood glucose data recovery icon appears during the blood glucose data recovery and disappears when the blood glucose data is fully recovered.

15. The drug delivery system with graphical user interface of claim 1, wherein
the main screen further includes a suspend infusion function icon, for displaying the state of hypoglycemia suspend infusion function or predicted hypoglycemia suspend infusion function in the drug infusion system.

16. The drug delivery system with graphical user interface of claim 1, wherein
the main screen further includes an alert status icon.

17. The drug delivery system with graphical user interface of claim 1 wherein
the detection device and the infusion device are provided in separate components.

18. The drug delivery system with graphical user interface of claim 1, wherein
the detection device and the infusion device are provided in same component.

19. A drug delivery system with graphical user interface, comprising:
a detection device, for detecting the blood glucose level of the user;
an infusion device, for infusing a drug into the user's body; and
a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein to access other screens by sliding on the main screen.

20. The drug delivery system with graphical user interface of claim 19, wherein
the sliding method includes the direction and/or the number of sliding, sliding in different directions and/or number of times to make the home screen to access other different screens.

21. The drug delivery system with graphical user interface of claim 20, wherein
the graphical user interface returns to the home screen from the other different screens by sliding.

22. The drug delivery system with graphical user interface of claim 21 wherein
the sliding method to access other different screens is related to the sliding method to return to the home screen from the other different screens.

23. The drug delivery system with graphical user interface of claim 22, wherein
the sliding method to access other different screens and the sliding method to return to the home screen from the other different screens has the same sliding number of times but different sliding directions.

24. The drug delivery system with graphical user interface of claim 20, wherein
the graphical user interface returns to the home screen from the other different screens by a particular way.

25. The drug delivery system with graphical user interface of claim 24, wherein
the user interface is set with a touch-sensitive button, the particular way is touching the touch-sensitive button to return to the home screen.

26. The drug delivery system with graphical user interface of claim 24, wherein
the particular way is tapping the user interface in succession to return to the home screen.

27. The drug delivery system with graphical user interface of claim 24, wherein
the particular way is sliding in a certain direction and number of times to return to the home screen.

28. The drug delivery system with graphical user interface of claim 24, wherein
the control device further includes a solid button, the particular way is pressing the solid button to return to the home screen.

29. The drug delivery system with graphical user interface of claim 19, wherein
the insulin information display area includes insulin infusion status information, IOB information and remaining insulin information in the infusion device, the user interface displaying the insulin infusion status information in a graphic and/or text manner.

30. The drug delivery system with graphical user interface of claim 19, wherein
the blood glucose information includes a blood glucose graphic display area and a status display area of the detection device.

31. The drug delivery system with graphical user interface of claim 30, wherein
the detection device status display area displays information that the detection device is in a general detection state or a special detection state.

32. The drug delivery system with graphical user interface of claim 30, wherein
the blood glucose graphical display area includes a blood glucose reading curve or blood glucose reading discrete points over a period of time, and the normal blood glucose value area or the standard lines for high blood glucose value and low blood glucose value.

33. The drug delivery system with graphical user interface of claim 31, wherein
when the detection device is in the general detection state, the detection device status display area further includes a blood glucose trend display area.

34. The drug delivery system with graphical user interface of claim 19, wherein
the main screen further includes a calibration display area of the detection device.

35. The drug delivery system with graphical user interface of claim 19 wherein
the main screen further includes a blood glucose data recovery icon, the blood glucose data recovery icon appears during the blood glucose data recovery and disappears when the blood glucose data is fully recovered.

36. The drug delivery system with graphical user interface of claim 19, wherein
the main screen further includes a suspend infusion function icon, for displaying the state of hypoglycemia suspend infusion function or predicted hypoglycemia suspend infusion function in the drug infusion system.

37. The drug delivery system with graphical user interface of claim 19, wherein
the main screen further includes an alert status icon.

38. A drug delivery system with graphical user interface, comprising:
a detection device, for detecting the blood glucose level of the user;
an infusion device, for infusing a drug into the user's body; and
a control device, wirelessly communicated with the detection device and the infusion device, the control device controlling the drug delivery of the infusion device, and the control device provided with a graphical user interface, the graphical user interface comprising a main screen, the main screen comprising an insulin information display area and a blood glucose information display area, wherein the insulin information display area includes insulin infusion status information, the blood glucose information display area includes a blood glucose graphical display area that graphically displays the user's blood glucose level in a first time period, and by user input, the graphical user interface displays the user's blood glucose level in other time periods.

39. The drug delivery system with graphical user interface of claim 38, wherein
the blood glucose level in other time periods is displayed on the main screen.

40. The drug delivery system with graphical user interface of claim 39, wherein
by the user input, a time period selection area is displayed on the main screen, by selecting different time periods, the blood glucose graphical display area displays blood glucose level in the same time period.

41. The drug delivery system with graphical user interface of claim 38 wherein
the blood glucose level in other time periods is displayed on an expanded blood glucose graphical display area.

42. The drug delivery system with graphical user interface of claim 41, wherein
the expanded blood glucose graphical display area at least partially cover the main screen.

43. The drug delivery system with graphical user interface of claim 42, wherein
the expanded blood glucose graphical display area includes at least a time period selection area and a blood glucose graphical display area.

44. The drug delivery system with graphical user interface of claim 43, wherein
the expanded blood glucose graphical display area further includes a blood glucose text display area, for displaying the information about the blood glucose value at the selected moment or special detection status information.

45. The drug delivery system with graphical user interface of claim 40 or 43, wherein
in response to another user input, the blood glucose graphical display area displays the information on blood glucose values at the selected moment or special detection status information.

46. The drug delivery system with graphical user interface of claim 43, wherein
the blood glucose graphic display area further includes an event indication icon to indicate the time the event occurs.

47. The drug delivery system with graphical user interface of claim 40 or 43, wherein
by another user input, the detailed blood glucose information interface is accessed from the blood glucose graphic display area.

48. The drug delivery system with graphical user interface of claim 47, wherein
the detailed blood glucose information interface at least includes basic statistics and distribution statistics.

49. The drug delivery system with graphical user interface of claim 38, wherein
the blood glucose information display area further includes the status display area of the detection device.

50. The drug delivery system with graphical user interface of claim 49, wherein
the detection device status display area displays information that the detection device is in a general detection state or a special detection state.

51. The drug delivery system with graphical user interface of claim 50, wherein
when the detection device is in the general detection state, the detection device status display area shows the blood glucose reading of the latest moment.

52. The drug delivery system with graphical user interface of claim 50, wherein
when the detection device is in the special detection state, the detection device status display area shows a text message with a specific meaning to alert the user that an abnormal blood glucose value was detected by the detection device or there is an abnormal in the detection device.

53. The drug delivery system with graphical user interface of any one of claim 49-52, wherein
the user interface displays the insulin infusion status information in a graphic and/or text manner.

54. The drug delivery system with graphical user interface of claim 53, wherein
the graphic is a ring with at least one color indicating the type and the progress of the insulin infusion, the text comprising an abbreviation of the type of infusion and the rate or the progress of the infusion.

55. The drug delivery system with graphical user interface of claim 54, wherein
the status display area of the detection device area is set inside the ring.

56. The drug delivery system with graphical user interface of claim 55, wherein
when the detection device is in the general detection state, the detection device status display area further includes a blood glucose trend display area.

57. The drug delivery system with graphical user interface of claim 56, wherein
the blood glucose trend display area is indicated by indicator arrows with different color, direction and number.

58. A drug delivery system for displaying drug infusion information, comprising:
a detection device, for detecting the blood glucose level of the user; and
a control device, wirelessly communicated with the infusion device, the control device controlling the drug delivery of the infusion device, and the control device provided with a graphical user interface, the graphical user interface comprising a main screen, the main screen comprising an insulin information display area, wherein the user interface displaying the insulin infusion status information in a graphic and/or text manner.

59. The drug delivery system for displaying drug infusion information of claim 58, wherein the graphic is a ring with at least one color indicating the type and the progress of the insulin infusion, the text comprising an abbreviation of the type of infusion and the rate or the progress of the infusion.

60. The drug delivery system for displaying drug infusion information of claim 59, wherein when the detection device is in the special detection state, the detection device status display area shows a text message with a specific meaning to alert the user that an abnormal blood glucose value was detected by the detection device or there is an abnormal in the detection device.

61. The drug delivery system for displaying drug infusion information of claim 60, wherein the main screen also includes a graphic display area of insulin infusion information, which displays insulin infusion information in graphical manner for a period of time.

62. The drug delivery system for displaying drug infusion information of claim 61, wherein in response to a first user input, the insulin infusion information graphic display area displays insulin infusion information at a selected moment.

63. The drug delivery system for displaying drug infusion information of claim 62, wherein in response to a second user input, the insulin infusion information graphic display area is accessed to an expanded insulin infusion information graphic display area.

64. The drug delivery system for displaying drug infusion information of claim 63, wherein the expanded insulin infusion information graphical display area includes at least a time period selection area and an insulin infusion information graphical display area.

65. The drug delivery system for displaying drug infusion information of claim 64, wherein the expanded blood glucose graphical display area further includes an insulin infusion information text display area.

66. The drug delivery system for displaying drug infusion information of claim 65, wherein the insulin infusion information graphic display area further includes an event indication icon to indicate the time the event occurs.

67. The drug delivery system for displaying drug infusion information of claim 66, wherein by a third user input, the insulin infusion information graphic display area is accessed to a detailed insulin infusion information interface.

68. The drug delivery system for displaying drug infusion information of claim 67, wherein the detailed insulin infusion information interface at least includes basic statistics.

69. The drug delivery system for displaying drug infusion information of claim 61, wherein in response to a fourth user input, a time period selection area is displayed on the main screen, by selecting different time periods to view the insulin infusion information level in the corresponding different time periods.

70. The drug delivery system for displaying drug infusion information of claim 69, wherein in response to a fifth user input, the detailed insulin infusion information interface is accessed from the insulin infusion information graphic display area.

71. The drug delivery system for displaying drug infusion information of claim 70, wherein the detailed insulin infusion information interface at least includes basic statistics.

72. The drug delivery system for displaying drug infusion information any one of claim 58-71, wherein
the main screen at least further includes one or more IOB icon, a remaining insulin amount icon, a last bolus infusion information icon and a remaining time icon of the infusion device.
